# EUROPÄISCHE PATENTSCHRIFT

(11) **EP 1 211 238 B1**
(45) Veröffentlichungstag und Bekanntmachung des Hinweises auf die Patenterteilung: **30.06.2004**
(21) Anmeldenummer: 01128321.5
(22) Anmeldetag: 29.11.2001
(51) Int. Cl.: C07C 213/08, C07C 213/04, C07C 213/00, C07C 215/12

(54) **Verfahren zur Herstellung von Ethanolaminen**
Process for the preparation of ethanolamines
Procédé pour la préparation d'éthanolamines

(30) Priorität: 01.12.2000 DE 10059629
(43) Veröffentlichungstag der Anmeldung: 05.06.2002
(73) Patentinhaber: BASF AKTIENGESELLSCHAFT, 67056 Ludwigshafen (DE)
(72) Erfinder: Melder, Johann-Peter, Dr., 67459 Böhl-Iggelheim (DE); Schulz, Gerhard, Dr., 67098 Bad Dürkheim (DE)
(74) Vertreter: Isenbruck, Günter, Dr.

(56) Entgegenhaltungen:
- EP-A- 0 673 920
- US-A- 4 328 370
- US-A- 5 693 866
- US-A- 6 063 965
- US-A- 6 075 168

## Beschreibung

Die Erfindung betrifft ein Verfahren zur Herstellung von Ethanolaminen (I) durch Konversion von von diesen verschiedenen Ethanolaminen (II).

Alkanolamine sind Basisprodukte für eine Vielzahl industrieller Anwendungen. Ethanolamine, wie Triethanolamin (TEA), oder deren Folgeprodukte werden beispielsweise in Waschflüssigkeiten für Gaswäschen, in Pflanzenschutzmitteln, Seifen, Waschmitteln und Shampoos sowie als Dispergiermittel und Emulgiermittel eingesetzt.

Ethanolamine können durch Hydroxyethylierung von Aminen durch Umsetzung der Amine mit Ethylenoxid hergestellt werden, siehe Ullmann's Encyclopedia of Industrial Chemistry, Kapitel Ethanolamines and Propanolamines - N-alkylated Ethanolamines.

Monoethanolamin (MEA), Diethanolamin (DEA) und Triethanolamin (TEA) werden durch Umsetzung von Ammoniak mit Ethylenoxid in flüssiger Phase unter Druck und bei erhöhter Temperatur, wie GB-A-760 215 oder EP-A-673 920 beschrieben, hergestellt.

Dabei fällt ein Ethanolamin-Gemisch an, das als Hauptkomponenten MEA, DEA und TEA enthält, das anschließend mehrstufig destillativ aufgearbeitet wird. Die Zusammensetzung des erhaltenen Ethanolamin-Gemischs kann verfahrensbedingt nur in engen Grenzen variiert werden. Bei der Herstellung eines Ethanolamins, beispielsweise von DEA, fallen also die anderen Ethanolamine (MEA, TEA) zwangsläufig als Koppelprodukte an. Besteht beispielsweise ein hoher Bedarf an DEA, so kann dieser unter Umständen nur befriedigt werden, indem eine Überproduktion der Koppelprodukte MEA und TEA in Kauf genommen wird.

Es hat daher nicht an Versuchen gefehlt, ein bestimmtes Ethanolamin durch Umwandlung anderer Ethanolamine zu erzeugen.

US-A-4,264,776 beschreibt die katalytische Oxidation von tertiären Aminen mit Sauerstoff zu sekundären Aminen in Gegenwart eines Aktivkohlekatalysators. Gemäß Beispiel 2 dieser Patentschrift wird TEA an Aktivkohle bei 115 °C mit 60 % Umsatz zu DEA mit unbestimmter Selektivität umgesetzt. Ein C₂-Rest des TEAs wird dabei oxidativ, z. B. zu Ameisensäure, abgebaut.

US 4,328,370 beschreibt die Umwandlung von niederen Trialkanolaminen zu Mono- und Dialkanolaminen durch Umsetzung mit Ammoniak bei erhöhter Temperatur in Gegenwart eines Hydrierkatalysators. Gemäß den Beispielen dieser Patentschrift kann TEA mit Ammoniak in Gegenwart von Pd/C oder Pd/Al₂O₃ und gegebenenfalls H₂ bei 200 bis 300 °C zu DEA und MEA umgesetzt werden. Die Selektivität der Bildung von DEA und MEA sinkt mit zunehmendem Umsatz. Die Gesamtausbeute an MEA und DEA (Summe) beträgt bis zu 50 % bei einem Umsatz von 66 %, und einer Selektivität von 79,7 %.

US 5,693,866 beschreibt die Behandlung von Alkanolaminen mit Alkalimetallhydroxiden zum Zweck der Verbesserung der Farbqualität der Alkanolamine, wobei die Alkanolamine chemisch nicht umgewandelt werden. In den Beispielen wird TEA mit NaOH bei Temperaturen von 165 bis 176 °C über eine Zeitdauer von 3 bis 65 h behandelt.

US 6,063,965 offenbart ein Verfahren zur Herstellung von Diethanolamin, bei dem aus Ethylenoxid und wässrigem Ammoniak eine erste Mischung enthaltend Wasser, Ammoniak, Monoethanolamin, Diethanolamin und Triethanolamin erhalten wird, und zu dieser Mischung Monoethanolamin und weiteres Ethylenoxid gegeben wird, wobei eine zweite Mischung enthaltend Monoethanolamin, Diethanolamin und Triethanolamin erhalten wird.

US 6,075,168 offenbart ein Verfahren zur Herstellung von Monoalkanolamin und Alkylenoxid in einer Reaktivdestillation.

EP-A 0 673 920 offenbart die Herstellung von Gemischen aus Mono-, Di- und Triethanolamin aus Ammoniak und Ethylenoxid in einem Rohrreaktor.

Der vorliegenden Erfindung lag die Aufgabe zugrunde, ein wirtschaftliches und effizientes Verfahren zur Herstellung eines oder mehrerer Ethanolamine (I) aus einem oder mehreren von den Ethanolaminen (I) verschiedenen Ethanolaminen (II) bereitzustellen, um überschüssig produzierte Ethanolamine (II) einer wirtschaftlich sinnvollen Verwertung zuzuführen.

Gelöst wird die Aufgabe durch ein Verfahren zur Konversion von Ethanolaminen, bei dem mindestens ein Ausgangsethanolamin (II) zu mindestens einem von dem Ausgangsethanolamin verschiedenen Zielethanolamin (I) umgesetzt wird, wobei Ausgangs- und Zielethanolamin ausgewählt sind aus der Gruppe bestehend aus Monoethanolamin, Diethanolamin und Triethanolamin, durch Behandlung des mindestens einen Ausgangsethanolamins (II) mit einer starken Base, ausgewählt aus der Gruppe bestehend aus basischen Alkali- und Erdalkalihydroxiden, Alkalialkoholaten und basischen Metalloxiden.

Das oder die Ethanolamine (I) können aus nur einem Ethanolamin (II) oder einem Gemisch mehrerer Ethanolamine (II) hergestellt werden.

So kann Diethanolamin durch Behandlung von reinem Monoethanolamin oder von reinem Triethanolamin mit einer starken Base erhalten werden. Monoethanolamin kann durch Behandeln von reinem Diethanolamin erhalten werden. Daneben entsteht im allgemeinen Triethanolamin.

Bevorzugt wird Diethanolamin durch Behandlung eines Gemischs enthaltend Monoethanolamin und Triethanolamin mit einer starken Base hergestellt. Bevorzugte Gemische enthalten TEA und MEA im Molverhältnis von 10 : 1 bis 1 : 10.

Die Behandlung des oder der Ethanolamine (I) kann in Gegenwart von Ammoniak durchgeführt werden. So kann Diethanolamin durch Behandlung eines Gemischs enthaltend Trimethanolamin und Ammoniak hergestellt werden. Daneben wird im allgemeinen Monoethanolamin erhalten. Bevorzugte Gemische enthalten TEA und Ammoniak im Molverhältnis von 10 : 1 bis 1 : 20.

Monoethanolamin kann ferner durch Behandeln eines Gemischs enthaltend Diethanolamin und Ammoniak erhalten werden.

Die genannten, in der Konversion eingesetzten Ethanolamingemische können weitere Ethanolamine enthalten. Es können auch komplexe Ethanolamingemische eingesetzt werden, die mehrere oder alle der oben aufgeführten Ethanolamine (II) enthalten. Das Ausgangsethanolamingemisch kann bereits zum Teil das oder die herzustellenden Ethanolamine (I) enthalten. Beispiele sind technische Ethanolamingemische, wie sie nach folgenden Verfahren erhalten werden können:
- durch Umsetzung von Ammoniak oder eines primären oder sekundären Amins mit Ethylenoxid, z. B. gemäß EP-A-673 920;
- durch 1,4-Addition von Ammoniak oder eines primären oder sekundären Amins an einen α,β-ungesättigten Aldehyd, wie Acrolein, und anschließende Reduktion, z. B. durch Hydrierung;
- durch 1,4-Addition von Ammoniak oder eines primären oder sekundären Amins an eine α,β-ungesättigte Säure, wie Acrylsäure, oder an einen α,β -ungesättigten Ester, wie Acrylsäureester, und anschließender Reduktion, z. B. durch Hydrierung;
- durch 1,4-Addition von Wasser an ein α,β-ungesättigtes Nitril, wie Acrylnitril, und anschließender Reduktion, z. B. durch Hydrierung;
- durch Aminierung von primären oder sekundären Alkoholen oder aminierende Hydrierung von Hydroxyaldehyden oder Hydroxyketonen.

N-(2-Aminoethyl)ethanolamin (AEEA) kann durch Umsetzung von Monoethanolamin oder Ammoniak mit Ethylenoxid in Gegenwart von Wasserstoff und einem Hydrier-, Dehydrier- oder Aminierungskatalysator erhalten werden.

Bevorzugt wird man von Ethanolamingemischen ausgehen, die das Wertprodukt (das herzustellende Ethanolamin (I)) nicht enthalten, da auch das Wertprodukt unter den Reaktionsbedingungen allmählich abgebaut werden kann.

Es wird vermutet, dass es unter Einwirkung starker Basen zu einer Spaltung von Ethanolaminen mit n 2-Hydroxyethylgruppen in das nächstniedrigere Ethanolamin mit n-1 2-Hydroxyethylgruppen und Ethylenoxid kommt. Das intermediär gebildete Ethylenoxid wird von einem Akzeptor, vorzugsweise Ammoniak oder Monoethanolamin, unter Bildung des entsprechenden nächsthöheren Ethanolamins abgefangen. Daher ist es erfindungsgemäß bevorzugt, ein Ethanolamin mit n 2-Hydroxyethylgruppen aus einem Gemisch eines Ethanolamins mit n+1 1 2-Hydroxyethylgruppen und eines Ethanolamins mit n-1 2-Hydroxyethylgruppen bzw. Ammoniak herzustellen. Ethylenoxid wird auch von den weiteren genannten Ethanolaminen (O,N,N-Tris(2-hydroxyethyl)ethanolamin, N-(2-Aminoethyl)ethanolamin, N-(2-Hydroxyethyl)piperazin, N-(2-Hydroxyethyl)morpholin und N,N'-Bis(2-hydroxyethyl)piperazin) freigesetzt, die im allgemeinen kein Wertprodukt darstellen. Diese können daher vorteilhafterweise ebenfalls im Ausgangsgemisch enthalten sein. Die im Eduktgemisch enthaltenen 2-Hydroxyethylamino-Einheiten (HO-CH₂-CH₂-N) bleiben insgesamt weitgehend erhalten.

Das erfindungsgemäße Verfahren ermöglicht es insbesondere, wirtschaftlich bedeutende Ethanolamine, wie MEA, DEA und TEA, ineinander zu überführen, insbesondere DEA aus MEA und/oder TEA herzustellen. Als Nebenprodukte entstehen bei dieser Reaktion Kupplungsprodukte wie N,N'-Bis(2-hydroxyethyl)piperazin, O-ethoxylierte Produkte wie O-(2-Hydroxyethyl)triethanolamin sowie höher ethoxylierte Produkte. Im Gegensatz zum Stand der Technik werden diese Produkte jedoch nicht verworfen, sondern können beispielsweise als Zementhilfsmittel verwendet werden.

Das oder die Ethanolamine (II) werden mit einer starken Base behandelt. Geeignet sind alle starken Basen, die in der Lage sind, Ethanolamine in nennenswertem Maße zu deprotonieren. Geeignete starke Basen sind basische Alkali- und Erdalkalihydroxide, Alkalialkoholate sowie basische Metalloxide wie Oxide der Seltenerdmetalle, beispielsweise LiOH, NaOH, KOH, CsOH, Ba(OH)₂, Ca(OH)₂, Natriummethanolat, Natriumethanolat, Kalium-t-butylat und Lanthanoxid. Die Basen können als Feststoff oder als wässrige oder alkoholische Lösung eingesetzt werden. Besonders bevorzugte starke Basen sind NaOH und KOH.

Das oder die Ethanolamine (II) können in einem inerten Lösungsmittel gelöst oder dispergiert vorliegen. Geeignete Lösungsmittel sind Alkohole wie Methanol, Ethanol, isoPropanol, n-Propanol, n-Butanol, 2-Ethylhexanol, Ether wie Tetrahydrofuran und 1,4-Dioxan, aliphatische und aromatische Kohlenwasserstoffe oder deren Gemische wie Pentan, Hexan, Heptan, Petrolether, Benzol, Toluol, Xylol, Mihagol und Wasser.

Das erfindungsgemäße Verfahren wird im allgemeinen bei Temperaturen oberhalb 180°C, bevorzugt bei 180 bis 270°C, besonders bevorzugt bei 190 bis 260°C, und insbesondere bei 200 bis 250°C in üblichen Reaktionsapparaten, wie Autoklaven, Rohrreaktoren oder Rührkessel, durchgeführt. Die Reaktionsdauer liegt bevorzugt zwischen 0,5 und 20 h und richtet sich nach der Zusammensetzung des Ausgangsgemischs, dem gewünschten Wertprodukt, der eingesetzten starken Base und der Reaktionstemperatur. Die Wahl dieser Parameter lässt sich vom Fachmann durch einige orientierende Versuche ohne weiteres optimieren. Zu lange Reaktionszeiten können die Ausbeute an Wertprodukt mindern, da auch das Wertprodukt unter den Reaktionsbedingungen der Spaltung unterliegen kann. Besonders bevorzugt ist es, das gebildete Wertprodukt, beispielsweise durch Destillation, kontinuierlich aus dem Reaktionsgemisch zu entfernen und es dadurch vor dem Abbau zu schützen.

Die Erfindung wird durch die nachstehenden Beispiele näher erläutert.

### Beispiele

### Umsetzung von TEA in Gegenwart von Ammoniak

### Vergleichsbeispiel V 1

60 g TEA und 30 g Ammoniak werden im Rührautoklaven ohne Zusatz einer basischen Verbindung 3 h lang bei 250 °C gerührt.

### Beispiel 1

60 g TEA, 27 g Ammoniak und 6 g KOH werden im Rührautoklaven 3 h lang bei 250 °C gerührt.

### Beispiel 2

60 g TEA, 32 g Ammoniak und 6 g NaOH werden im Rührautoklaven 3 h lang bei 250 °C gerührt.

Anschließend wird eine Probe aus dem Reaktorraum entnommen, abgekühlt, mit Trifluoressigsäureanhydrid derivatisiert und gegen Diethylenglykoldimethylether als internen Standard quantitativ gaschromatographisch analysiert.

Die Ergebnisse der Versuche sind in Tabelle 1 zusammengefasst.

**Tabelle 1**

| Beispiel | MEA [Gew.-%] | DEA [Gew.-%] | DIHEP* [Gew.-%] | TEA-Ether** [Gew.-%] | TEA-Umsatz [%] | MEA-Selektivität [mol%] | DEA-Selektivität [mol%] |
|---|---|---|---|---|---|---|---|
| V 1 | 0,54 | 1,69 | 0,00 | 0,74 | 10,3 | 12,6 | 23,2 |
| 1 | 6,08 | 20,86 | 0,87 | 1,21 | 84,7 | 17,5 | 35,0 |
| 2 | 12,56 | 10,69 | 3,67 | 0,67 | 95,7 | 21,1 | 19,7 |

| | | | | | | | |
|---|---|---|---|---|---|---|---|
| * N,N'-Bis(2-hydroxyethyl)piperazin | | | | | | | |
| ** O-(2-Hydroxyethyl)triethanolamin | | | | | | | |

Man erkennt gegenüber dem Vergleichsbeispiel, dass in Gegenwart von NaOH bzw. KOH die niederen Ethanolamine MEA und DEA bei deutlich gesteigerten Umsätzen und mit zumindest vergleichbarer Selektivität erhalten werden.

### Umsetzung von TEA und MEA

### Allgemeine Arbeitsvorschrift

In einem 50 ml-Rührautoklaven werden, wenn nicht anders angegeben, 20 g TEA (0,13 mol) und 10 g MEA (0,16 mol) mit 2 g der angegebenen stark basischen Verbindung vorgemischt und während der angegebenen Zeit bei der angegebenen Temperatur gerührt. Anschließend wird eine Probe aus dem Reaktorraum entnommen, abgekühlt, mit Trifluoressigsäureanhydrid derivatisiert und gegen Diethylenglykoldimethylether als internen Standard quantitativ gaschromatographisch analysiert.

In den nachfolgenden Beispielen wurde abweichend von der allgemeinen Arbeitsvorschrift gearbeitet.

### Vergleichsbeispiel V 2

Das MEA/TEA-Gemisch wird ohne Zusatz einer basischen Verbindung erhitzt.

### Beispiel 3

30 g TEA werden mit 2 g KOH erhitzt.

### Beispiel 4

30 g MEA werden mit 2 g KOH erhitzt.

### Beispiel 13

24 g TEA und 6 g MEA werden mit 2,4 g KOH erhitzt.

### Beispiel 19

Das MEA/TEA-Gemisch wird mit 2 g NaOH gelöst in 2 g Wasser erhitzt.

### Beispiel 20

Das MEA/TEA-Gemisch wird mit 2 g NaOH gelöst in 5 g Ethanol erhitzt.

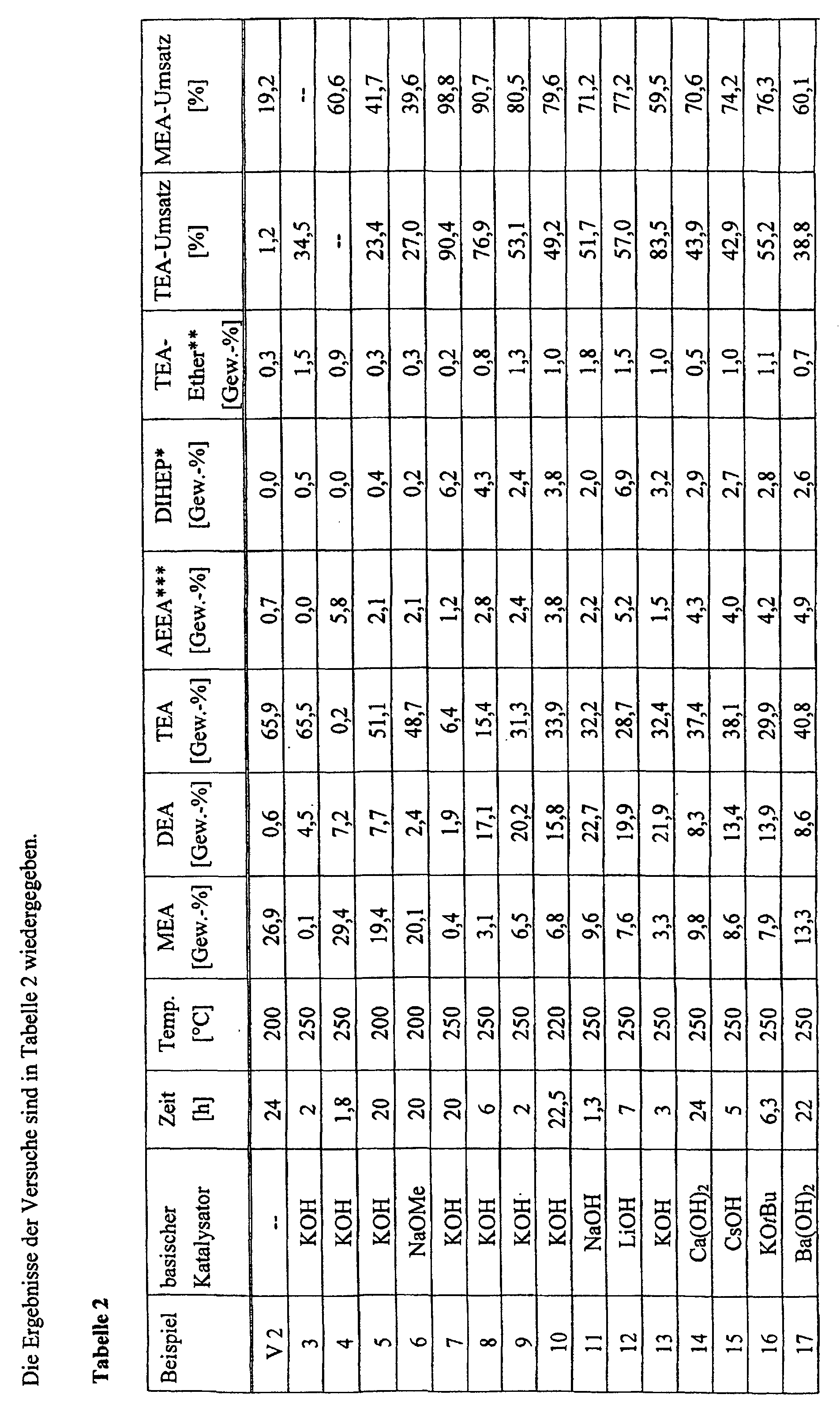

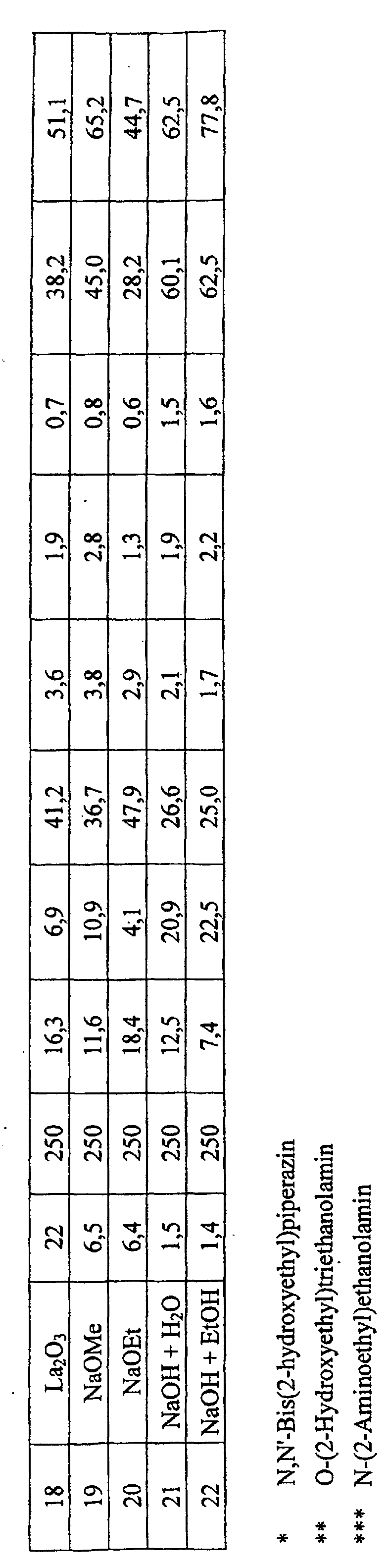

Aus den Beispielen geht hervor, dass
- die Zugabe eines Akzeptors, wie Ammoniak oder MEA, die Ausbeute an Ethanolamin (I) erhöht (Beispiele 3, 4 und 9);
- Reaktionsdauer und -temperatur auf die basische Verbindung abzustimmen sind (Beispiele 5 bis 22);
- die Umsetzung bevorzugt bei einem Teilumsatz der Ausgangsethanolamine (MEA und TEA) abgebrochen wird, da das gebildete Wertprodukt (DEA) abgebaut wird (Beispiele 7 bis 9);
- die basische Verbindung auch als Lösung zugesetzt werden kann (Beispiele 21 und 22);
- die Konversion nicht nur von höheren zu niedrigeren Ethanolaminen, sondern auch umgekehrt verläuft (Beispiele 3 und 4).

## Patentansprüche

1. Verfahren zur Konversion von Ethanolaminen, bei dem mindestens ein Ausgangsethanolamin (II) zu mindestens einem von dem Ausgangsethanolamin verschiedenen Zielethanolamin (I) umgesetzt wird, wobei Ausgangs- und Zielethanolamin ausgewählt sind aus der Gruppe bestehend aus Monoethanolamin, Diethanolamin und Triethanolamin, durch Behandlung des mindestens einen Ausgangsethanolamins (II) mit einer starken Base, ausgewählt aus der Gruppe bestehend aus basischen Alkali- und Erdalkalihydroxiden, Alkalialkoholaten und basischen Metalloxiden.

2. Verfahren nach Anspruch 1, **dadurch gekennzeichnet, dass** das mindestens eine Ausgangsethanolamin (II) in Gegenwart von Ammoniak mit der starken Base behandelt wird.

3. Verfahren nach Anspruch 1 oder 2, **dadurch gekennzeichnet, dass** das Ausgangsethanolamin in Gegenwart einer Verbindung, ausgewählt aus der Gruppe bestehend aus O,N,N Tris(2-hydroxyethyl)ethanolamin, N-(2-Aminoethyl)ethanolamin, N-(2-Hydroxyethyl)piperazin, N-(2-Hydroxyethyl)morpholin und N,N'-Bis-(2-hydroxyethyl)piperazin, mit der starken Base behandelt wird.

4. Verfahren nach Anspruch 1, **dadurch gekennzeichnet, dass** das Ausgangsethanolamin Monoethanolamin und das Zielethanolamin Diethanolamin ist.

5. Verfahren nach Anspruch 2, **dadurch gekennzeichnet, dass** das Ausgangsethanolamin Triethanolamin und das Zielethanolamin Diethanolamin ist.

6. Verfahren nach Anspruch 1, **dadurch gekennzeichnet, dass** das Ausgangsethanolamin ein Gemisch aus Monoethanolamin und Triethanolamin und das Zielethanolamin Diethanolamin ist.

7. Verfahren nach Anspruch 2, **dadurch gekennzeichnet, dass** das Ausgangsethanolamin Triethanolamin und das Zielethanolamin ein Gemisch aus Monoethanolamin und Dimethanolamin ist.

8. Verfahren nach Anspruch 7, wobei man als starke Base Natriumhydroxid oder Kaliumhydroxid einsetzt.

9. Verfahren nach Anspruch 7 oder 8, wobei man die starke Base als wässrige oder alkoholische Lösung einsetzt.

10. Verfahren nach einem der Ansprüche 1 bis 9, wobei das oder die Ethanolamine in einem inerten Lösungsmittel gelöst vorliegen.

11. Verfahren nach einem der Ansprüche 1 bis 10, wobei die Behandlung bei 180°C bis 270°C über einen Zeitraum von 0,5 h bis 20 h durchgeführt wird.

## Claims

1. A process for the conversion of ethanolamines in which at least one starting ethanolamine (II) is converted to at least one target ethanolamine (I) different from the starting ethanolamine, where starting and target ethanolamine are chosen from the group consisting of monoethanolamine, diethanolamine and triethanolamine, by treating the at least one starting ethanolamine (II) with a strong base chosen from the group consisting of basic alkali metal and alkaline earth metal hydroxides, alkali metal alkoxides and basic metal oxides.

2. A process as claimed in claim 1, wherein the at least one starting ethanolamine (II) is treated with the strong base in the presence of ammonia.

3. A process as claimed in claim 1 or 2, wherein the starting ethanolamine is treated with the strong base in the presence of a compound chosen from the group consisting of O,N,N-tris(2-hydroxyethyl)ethanolamine, N-(2-aminoethyl)ethanolamine, N-(2-hydroxyethyl)piperazine, N-(2-hydroxyethyl)-morpholine and N,N'-bis(2-hydroxyethyl)piperazine.

4. A process as claimed in claim 1, wherein the starting ethanolamine is monoethanolamine and the target ethanolamine is diethanolamine.

5. A process as claimed in claim 2, wherein the starting ethanolamine is triethanolamine and the target ethanolamine is diethanolamine.

6. A process as claimed in claim 1, wherein the starting ethanolamine is a mixture of monoethanolamine and triethanolamine and the target ethanolamine is diethanolamine.

7. A process as claimed in claim 2, wherein the starting ethanolamine is triethanolamine and the target ethanolamine is a mixture of monoethanolamine and dimethanolamine.

8. A process as claimed in claim 7, wherein sodium hydroxide or potassium hydroxide is used as strong base.

9. A process as claimed in claim 7 or 8, wherein the strong base is used in the form of an aqueous or alcoholic solution.

10. A process as claimed in any of claims 1 to 9, wherein the ethanolamine or ethanolamines are present in dissolved form in an inert solvent.

11. A process as claimed in any of claims 1 to 10, wherein the treatment is carried out at 180°C to 270°C over a period of from 0.5 h to 20 h.

## Revendications

1. Procédé pour la conversion d'éthanolamines, dans lequel au moins une éthanolamine de départ (II) est convertie en au moins une éthanolamine cible (I) différente de l'éthanolamine de départ, l'éthanolamine de départ et l'éthanolamine cible étant choisies parmi le groupe constitué de la monoéthanolamine, de la diéthanolamine et de la triéthanolamine, par traitement d'au moins une éthanolamine de départ (II) avec une base forte, choisie parmi le groupe constitué des hydroxydes d'alcalins et d'alcalino-terreux basiques, des alcoolats d'alcalins et des oxydes métalliques basiques.

2. Procédé selon la revendication 1, **caractérisé en ce que** la moins une éthanolamine de départ (II) est traitée avec la base forte en présence d'ammoniac.

3. Procédé selon la revendication 1 ou 2, **caractérisé en ce que** l'éthanolamine de départ est traitée avec une base forte en présence d'un composé choisi parmi le groupe constitué de la O,N,N-tris(2-hydroxyéthyl)éthanolamine, N-(2-aminoéthyl)éthanolamine, N-(2-hydroxyéthyl)pipérazine, N-(2-hydroxyéthyl)morpholine et N,N'-bis-(2-hydroxyéthyl)pipéra-zine.

4. Procédé selon la revendication 1, **caractérisé en ce que** l'éthanolamine de départ est la monoéthanolamine et l'éthanolamine cible est la diéthanolamine.

5. Procédé selon la revendication 2, **caractérisé en ce que** l'éthanolamine de départ est la triéthanolamine et l'éthanolamine cible est la diéthanolamine.

6. Procédé selon la revendication 1, **caractérisé en ce que** l'éthanolamine de départ est un mélange de monoéthanolamine et de triéthanolamine et l'éthanolamine cible est la diéthanolamine.

7. Procédé selon la revendication 2, **caractérisé en ce que** l'éthanolamine de départ est la triéthanolamine et l'éthanolamine cible est un mélange de monoéthanolamine et diéthanolamine.

8. Procédé selon la revendication 7, où l'on utilise comme base forte de l'hydroxyde de sodium ou de l'hydroxyde de potassium.

9. Procédé selon la revendication 7 ou 8, où l'on utilise la base forte sous forme de solution aqueuse ou alcoolique.

10. Procédé selon l'une quelconque des revendications 1 à 9, où la ou les éthanolamines sont présentes dissoutes dans un solvant inerte.

11. Procédé selon l'une quelconque des revendications 1 à 10, où le traitement est réalisé pendant une durée d'une demie heure à 20 h entre 180°C et 270°C.
